# EUROPEAN PATENT APPLICATION

(11) **EP 1 291 033 A1**
(43) Date of publication of application: **12.03.2003**
(21) Application number: 01919907.4
(22) Date of filing: 12.04.2001
(51) Int. Cl.: A61M 25/00, A61B 1/00

(54) **TUBE FOR MEDICAL CARE AND METHOD FOR PREPARING THE SAME**

(30) Priority: 15.05.2000 JP 2000141632
(71) Applicant: KANAGAWA ACADEMY OF SCIENCE AND TECHNOLOGY, Kanagawa 213-0012 (JP); JAPAN SCIENCE AND TECHNOLOGY CORPORATION, Kawaguchi-shi, Saitama 332-0012 (JP)
(72) Inventor: KUBOTA, Yoshinobu, Kanazawa-ku, Yokohama-shi, Kanagawa 236- (JP); FUJISHIMA, Akira, Nakahara-ku, Kawasaki-shi, Kanagawa 211- (JP); HASHIMOTO, Kazuhito, Sakae-ku, Yokohama-shi, Kanagawa 244-084 (JP); WATANABE, Toshiya, Fujisawa-shi, Kanagawa 251-0022 (JP); OHKO, Yoshihisa, Toshima-ku, Tokyo 170-0011 (JP); NIWA, Chisa, Konan-ku,Yokohama-shi.Kanagawa 233-0003 (JP)
(74) Representative: Kador & Partner
(86) International application number: JP0103159
(87) International publication number: WO01087397

(57) **Abstract**

A tube for medical care comprises a tube body comprising an elastomer and a photocatalyst layer containing titanium oxide and having a great number of microcracks is formed on the surface of the tube body. The tube retains elasticity, and also can secure the close contact of the elastomer surface with the photocatalyst layer and thus has a titanium oxide photocatalyst layer fixed firmly to the tube surface, due to the stress relaxation by the great number of microcracks. The tube exhibits excellent antibacterial activity due to the photocatalyst layer. The tube exhibits more excellent antibacterial activity, when another antibacterial substance is carried on the photocatalyst layer.

## Description

### Technical Field of the Invention

The present invention relates to a tube for medical care and a method for preparing the tube, and specifically to a tube for medical care possessing an antibacterial activity by using a photocatalyst containing titanium oxide and a method for preparing the tube.

### Background Art of the Invention

Recently, the photocatalytic reaction of titanium oxide has been paid attention to, and it has been known that the photocatalytic reaction of titanium oxide is remarkably effective for decomposition of various organic substances and remarkably effective also for giving an antibacterial activity such as prevention of adhesion or sterilization of a bacillus or a virus.

In the field of medical care, it is preferred to give an antibacterial activity to substantially all devices and all materials around the devices, and in particular, an excellent antibacterial activity is required for various tubes for medical care, which are used directly for medical treatment, etc. (for example, a catheter to be inserted into or placed in a human body, or an endoscope), from the viewpoint of prevention of secondary infection.

Therefore, it is considered that it is quite desirable to be able to give an antibacterial activity to such tubes for medical care by providing a titanium oxide photocatalyst layer. However, a tube for medical care is usually made as a flexible tube having a flexibility or an elasticity such as a silicone rubber tube, and from the reason that it is difficult to form and fix a thin titanium oxide photocatalyst layer, which is formed as a substantially hard inorganic substance film, on the surface of such a flexible tube, there is no proposal at present for providing a titanium oxide photocatalyst layer onto a tube for medical care.

The photocatalyst layer is formed mainly by titanium oxide particles, and a binder is used to fix the particles onto a tube by coating. Usually, in order to provide a flexibility to a coating layer, a component containing a large amount of organic substances is employed as the binder. In a case of coating of a photocatalyst, however, because the photocatalyst decomposes organic substances by oxidation, it is necessary to use an inorganic substance as the binder. However, such an inorganic substance is hard and brittle similarly to titanium oxide of photocatalyst. Although most frequently used binder for photocatalyst is silica, it also has a problem of hardness and brittleness.

Therefore, there has been a problem that a coating layer formed with such a hard and brittle component cannot follow the motion of a substrate causing a great expansion such as an elastomer, thereby being released away.

### Disclosure of the Invention

Accordingly, an object of the present invention is to provide a high-reliability tube for medical care in which a photocatalyst layer is firmly fixed onto the surface of a tube body comprising an elastomer with a flexibility, and a method for preparing the same.

To accomplish the above object, a tube for medical care according to the present invention comprises a tube body comprising an elastomer, and a photocatalyst layer formed on the surface of the tube body which contains titanium oxide and has a great number of microcracks.

A method for preparing a tube for medical care according to the present invention comprises the steps of treating the surface of a tube body comprising an elastomer with an acid; and forming a photocatalyst layer containing titanium oxide on the treated surface of the tube body by coating; and at the same time, generating a great number of microcracks in the photocatalyst layer.

Although the titanium oxide photocatalyst layer may be formed directly on the surface of the tube body comprising an elastomer, in order to increase the adhesive strength, it is preferred to employ a constitution wherein an adhesive layer is interposed between at least one of inner and outer surfaces of the tube body and the titanium oxide photocatalyst layer, and the titanium oxide photocatalyst layer and the adhesive layer are formed substantially as an integral layer.

In this tube for medical care, a structure may be employed wherein a light-guiding material, extending in the longitudinal direction of the tube body and capable of guiding a light toward the titanium oxide photocatalyst layer, is buried in the tube body. Although the material of the tube body comprising an elastomer is not particularly restricted, because most of the present tube bodies are made of a silicone rubber, also in the present invention, a silicone rubber can be used.

The tube for medical care and the method for preparing the same according to the present invention typically can be applied to various catheters aimed to be placed in a human body when inserted into the human body, or various endoscopes, but not limited thereto, the present invention can be applied to any type of tube for medical care for which an antibacterial activity is desired to be given.

In the present invention, in order to achieve the aforementioned object, by introducing a great number of microcracks into the photocatalyst layer containing titanium oxide, even when an elastomer, which is a substrate, is expanded/contracted, stress relaxation can be achieved by the microcracks, and the close contact of the elastomer surface with the photocatalyst layer can be prevented from being lost.

Although generally it is considered that cracks of a film may cause release of the film, the inventors of the present invention have found that, in a case where a hard coating is carried out, the stress applied to the thin film divided into fine parts, in a condition where the close contact in an interface with a substrate is maintained, is relaxed at the crack portions, and therefore, the close contact can be maintained rather well, and from this knowledge the present invention has been completed.

The microcracks in the present invention must be fine to an extent capable of sufficiently relaxing the stress. Further, in this case, if the film is too thin, cracks are difficult to be generated, and if too thick, cracks break the interface when the cracks propagate, and therefore, it is preferred to control the thickness of the film within an adequate range. An optimum thickness of the film is in the range of 10 nm to 10 µm.

In such a tube for medical care according to the present invention, when observed by a microscope, it was actually recognized that a great number of microcracks were generated in the titanium oxide photocatalyst layer formed on the surface of a tube body comprising an elastomer.

Further, in the tube for medical care according to the present invention, it is effective that another antibacterial substance in addition to titanium oxide is carried on the titanium oxide photocatalyst layer. The antibacterial substance may be carried on the surface of the titanium oxide photocatalyst layer, and the antibacterial substance may be carried on each of the titanium oxide particles and the particles deposited with the antibacterial substance may be coated to form the photocatalyst layer. In the latter case, because the interior of the photocatalyst layer itself is formed by gathering particles coated with the antibacterial substance, there is an advantage that the layer can be fixed more firmly as compared with a case where the antibacterial substance is coated only on the surface of the photocatalyst layer. As the antibacterial substance, for example, silver can be used, and other antibacterial substances can also be used.

In the above-described tube for medical care and the method for preparing the tube according to the present invention, a practicable and sufficiently strong titanium oxide photocatalyst layer having a great number of microcracks is formed on the surface of the tube body comprising an elastomer and possessing a flexibility, and by irradiating light thereto, the tube for medical care having the titanium oxide photocatalyst layer can exhibit excellent antibacterial activity, as shown in the examples described later. By this excellent antibacterial activity, not only can sterilization be achieved, but also, adhesion of a bacillus or a virus can be prevented, and therefore, problems such as secondary infection when a tube for medical care is used can be effectively solved.

Furthermore, when an antibacterial substance in addition to titanium oxide is carried on the titanium oxide photocatalyst layer, an antibacterial activity due to the antibacterial substance is added, a further excellent antibacterial activity can be obtained as a whole. For example, when an antibacterial substance such as silver is carried on the layer, an excellent antibacterial activity can be given even in the dark.

### Brief explanation of the drawings

Figure 1 is a schematic perspective view of a catheter as a tube for medical care according to an embodiment of the present invention.

Figure 2 is a schematic view of a device showing a dip coating method.

Figure 3 is a schematic view showing a method for carrying an antibacterial substance on the surface of a titanium oxide photocatalyst layer.

Figure 4 is a schematic view showing another method for carrying an antibacterial substance on the surface of a titanium oxide photocatalyst layer.

Figure 5 is a graph showing the property of pigment decomposition of a titanium oxide photocatalyst layer.

Figure 6 is a cross-sectional view of a sample of a catheter with a titanium oxide photocatalyst layer.

Figure 7 is a perspective view showing a section of catheter after cutting and opening the sample depicted in Fig. 6.

Figure 8 is a graph showing light transmittance of catheters.

Figure 9 is a schematic perspective view showing a method for determining the antibacterial activity of a catheter.

Figure 10 is a graph showing the result of the examination for determining antibacterial activities of catheters.

Figure 11 is a graph showing the result of the examination for determining antibacterial activities of other catheters.

Figure 12 is a graph showing the result of the examination for determining antibacterial activities of further other catheters.

Figures 13A and 13B are cross-sectional views of catheters with light-guiding materials.

Figure 14 is a view of a surface of a titanium oxide photocatalyst layer deposited with silver, observed by an electron microscope.

Figure 15 is a view of the surface of the titanium oxide photocatalyst layer shown in Fig. 14, observed by an electron microscope with an increased magnification.

Figure 16 is a graph showing the result of an examination for determining antibacterial activities in a case where silver is carried on the layer.

Figure 17 is a graph showing the result of another examination for determining antibacterial activities in a case where silver is carried on the layer.

Figure 18 is a graph showing the result of an examination for confirming the effect due to the carried silver with respect to antibacterial activity.

Figure 19 is a graph showing the result of an examination for comparing the antibacterial activities between a catheter deposited with silver according to the present invention and a catheter adhered with silver on the market.

Figure 20 is a graph showing the antibacterial activity in a case where silver is carried on the inside of a titanium oxide photocatalyst layer.

### The best mode for carrying out the Invention

Hereinafter, desirable embodiments of the present invention will be explained referring to the figures.

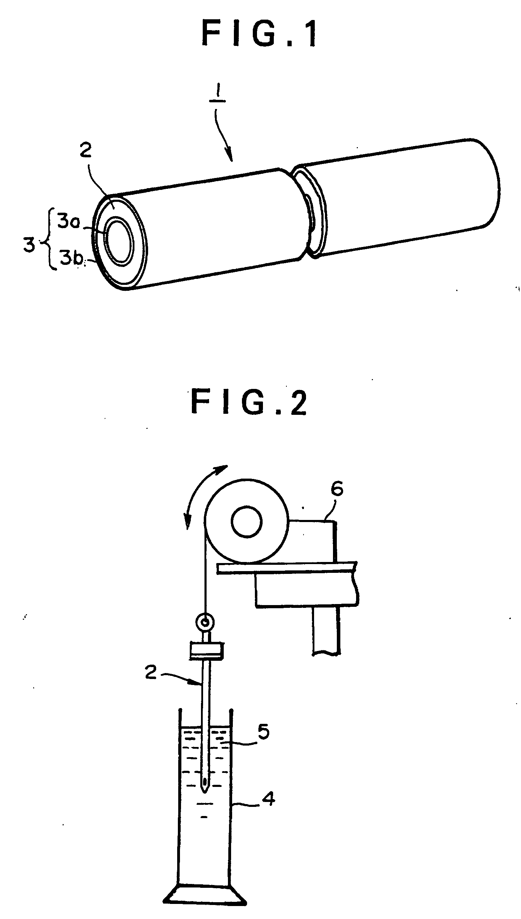

Fig. 1 is a partial perspective view of a tube for medical care according to an embodiment of the present invention, and shows a case where the present invention is applied to a catheter. In Fig. 1, numeral 1 indicates a catheter as a tube for medical care, and catheter 1 comprises a tube body 2 comprising an elastomer and a titanium oxide photocatalyst layer 3 formed on the surface of the tube body. In this embodiment, titanium oxide photocatalyst layer 3 is formed on both inner and outer surfaces of tube body 2 (layers 3a, 3b).

The above-described catheter 1 is prepared as follows, for example.

First, after tube body 2, particularly, the surface thereof, is cleaned and dried, the surface is treated with an acid. The acid used for the acid treatment is not particularly limited, and for example, sulfuric acid can be used. The treatment is carried out, for example, by immersing tube body 2 in an acid controlled at a predetermined concentration for a predetermined time.

After the acid treatment, a titanium oxide photocatalyst layer is formed by coating on an object surface of tube body 2, that is, on at least one of the inner or outer surfaces of the tube body, and fixed thereto. Although it is possible that this coating of the titanium oxide photocatalyst layer is performed directly onto the acid treated surface of tube body 2, for example, a method may be employed wherein, using a sol solution of a low-temperature curing two-layer type, at first an adhesive layer is coated and dried, and thereonto a titanium oxide photocatalyst layer is coated, and a stronger layer can be formed by this method. Even in such a case where an adhesive layer and a titanium oxide photocatalyst layer are coated in order, the completed layer is formed as an integral titanium oxide photocatalyst layer 3.

Although a method of spray and the like may be employed as the coating method, in order to form a uniform and thin titanium oxide photocatalyst layer, a dip coating method is preferably employed. For example, as shown in Fig. 2, a dipping solution 5 to be coated is stored in a container 4, tube body 2 after acid treatment is soaked in the dipping solution 5 for a predetermined time, and thereafter, the coated tube body 2 is pulled up at a predetermined speed by controlling the rotational speed of a motor 6. In this coating, since the temperature of dipping solution 5, the time for soaking, the speed for pulling up, the temperature for drying after pulling up, and the time for drying become important factors to decide the condition for forming the titanium oxide photocatalyst layer, optimum conditions may be decided by various examinations, depending upon the material of tube body 2 and the kind of the dipping solution 5.

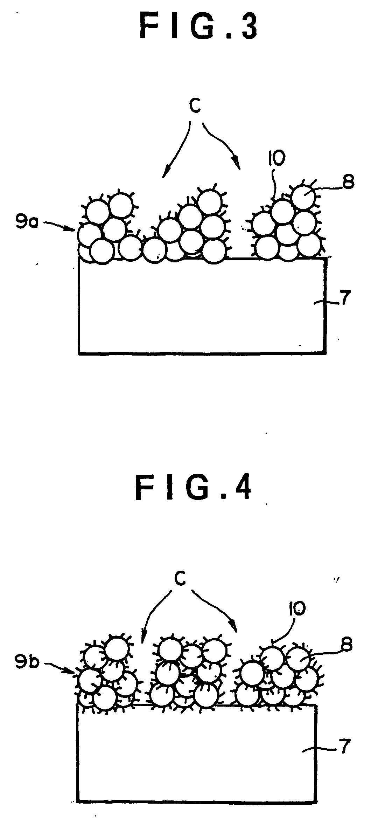

Further, an antibacterial substance in addition to titanium oxide, especially, an antibacterial metal, for example, silver, can be carried on the titanium oxide photocatalyst layer. In this case, the antibacterial substance may be carried on the surface of the titanium oxide photocatalyst layer by coating, and the antibacterial substance may be carried on titanium oxide particles forming the photocatalyst layer in advance, and using the titanium oxide particles carried with the antibacterial substance, the photocatalyst layer may be formed by coating. In the former method, as shown in Fig. 3 for example, a photocatalyst layer 9a mainly comprising titanium oxide particles 8 is formed on the surface of a substrate 7 comprising an elastomer which forms a tube body, and an antibacterial substance 10 is coated on the surface of the photocatalyst layer 9a. Label C shows microcracks formed. In the latter method, as shown in Fig. 4, for example, the antibacterial substance 10 is carried on each of the titanium oxide particles 8 in advance, and because a photocatalyst layer 9b is formed by the titanium oxide particles 8 carried with the antibacterial substance 10, the photocatalyst layer 9b is formed in a structure where the antibacterial substance 10 is dispersed into the interior of the layer, and therefore, the antibacterial substance 10 is carried and fixed more securely. Label C shows microcracks formed.

### Examples

Hereinafter, the present invention will be explained more concretely based on examples, and the results of determination with respect to the properties of the tube for medical care according to the present invention, particularly, with respect to antibacterial activity, will also be explained.

A conventional catheter (made of a silicone rubber, an all silicone balloon catheter of a kidney pelvis type) was prepared as a tube body, and after it was cleaned and dried, the surface thereof was treated with acid by soaking it in 5M sulfuric acid for 3 hours.

The tube body, the surface of which had been acid treated, was dip coated once in an adhesive for a photocatalyst layer "NDC-100A" (produced by Nihon Soda Corporation) at a condition of room temperature and a relative humidity of 30%, the tube body was pulled up at a speed of 15 cm/min., and dried at a temperature of 110 °C for 15 minutes to form adhesive layers on the inner and outer surfaces of the tube body.

Then, the tube body formed with the adhesive layers was dip coated once in a sol solution of titanium oxide "NDC-110C" (produced by Nihon Soda Corporation) at a condition of room temperature and a relative humidity of 30%, the tube body was pulled up at a speed of 15 cm/min., and dried at a temperature of 110 °C for 15 minutes to form titanium oxide photocatalyst layers on the inner and outer surfaces;of the tube body (the film thickness of the formed titanium oxide photocatalyst layer: 1.5 µm). It was recognized by observation that the adhesive layers and the titanium oxide photocatalyst layers were both coated uniformly.

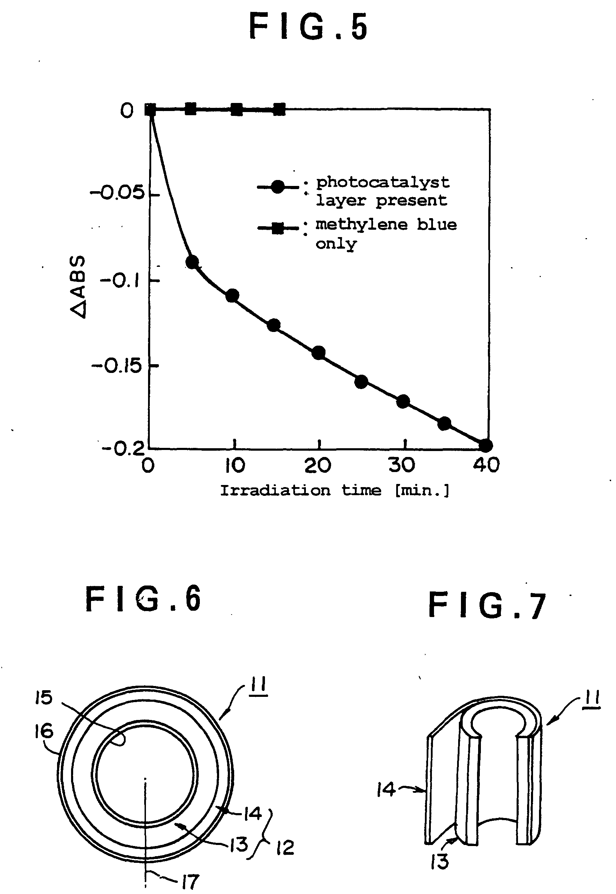

With respect to the prepared catheter with the titanium oxide photocatalyst layers, first, it was confirmed that the titanium oxide photocatalyst layers had photocatalytic activity. The confirmation was carried out as follows. Using methylene blue as a pigment, at a condition where the pigment was applied onto the surface of the catheter, ultraviolet rays were used to irradiate the titanium oxide photocatalyst layer of the catheter at an intensity of about 1 mW/cm², and the degree of the decomposition of methylene blue relative to the irradiation time was determined by a color-difference meter. The property for the pigment decomposition was indicated as the degree of Δ ABS of the color-difference meter. As a result, as shown in Fig. 5, in a case where the ultraviolet ray was directly irradiated only on the methylene blue, substantially no property for the pigment decomposition was exhibited. However, in a case where the ultraviolet rays were irradiated on the methylene blue applied on the catheter with the titanium oxide photocatalyst layer, the methylene blue was decomposed to an extent proportional with the irradiation time of the ultraviolet rays, and it was recognized that the titanium oxide photocatalyst layer formed on the catheter had an excellent photocatalytic activity.

Next, the transmittance of ultraviolet ray of the above-described catheter with the titanium oxide photocatalyst layer was determined as to various wavelengths. An ultraviolet ray transmittance measuring device ("UV-VIS") was used for the determination, and particularly, the determination was carried out paying attention to wavelengths less than 380 nm, which is an excitation wavelength for exhibiting the photocatalytic activity of the above-described titanium oxide photocatalyst layer, especially, a wavelength around 360 nm. The above-described catheter with the titanium oxide photocatalyst layer was cut and opened, and was set on a sample table of the measuring device.

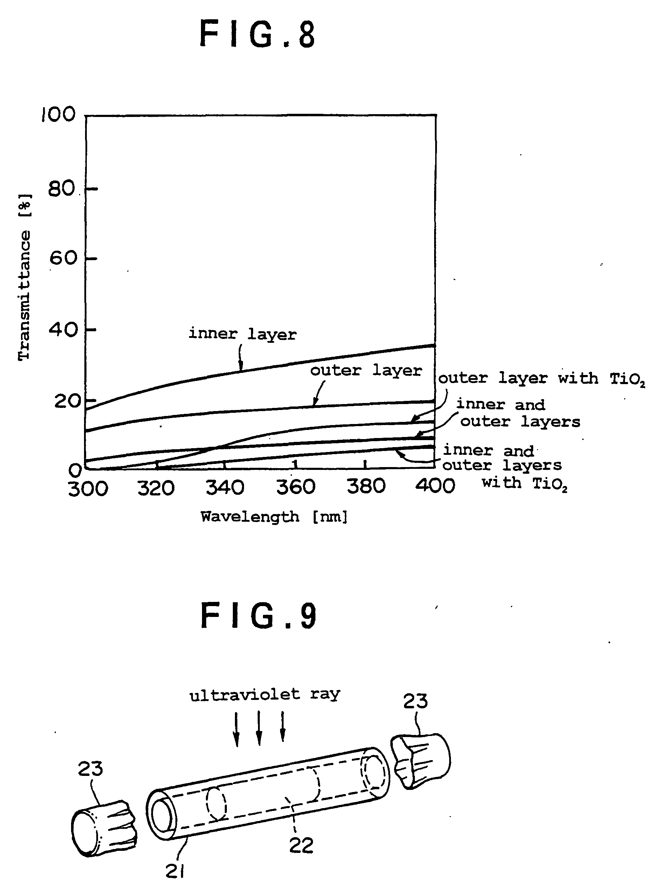

Namely, as shown in Figs. 6 and 7, a sample 11 was prepared by cutting the catheter with the titanium oxide photocatalyst layer at an appropriate length. When the cross section of this sample 11 was observed, the catheter portion 12 of the kidney pelvis-type all silicone balloon catheter as a tube body comprised a transparent inner layer 13 and a whitish outer layer 14, and titanium oxide photocatalyst layers 15, 16 were formed on the inner surface of the inner layer 13 and the outer surface of the outer layer 14, respectively. As shown in Fig. 6, the cylindrical sample 11 was cut along a cutting line 17 extending in the axis direction and opened in a plate-like form. Further, a sample comprising only the catheter portion 12 without formed titanium oxide photocatalyst layers was prepared similarly. In each of these samples, since the inner layer 13 and the outer layer 14 could be peeled from each other as shown in Fig. 7, the following various plate-like samples were prepared, and the transmittance of the ultraviolet rays was determined. The result is shown in Fig. 8.

In Fig. 8, "inner and outer layers" means a plate-like sample prepared by cutting and opening the sample comprising only catheter portion 12, "outer layer" means a plate-like sample prepared by peeling the whitish film of the above-described sample and determining the peeled film, "inner layer" means a plate-like sample of the transparent catheter portion left after peeling the outer whitish film and determining the transparent portion, "inner and outer layers with TiO₂" means a plate-like sample prepared by cutting and opening the catheter with the titanium oxide photocatalyst layers sample and determining the cut and opened sample as it was, and "outer layer with TiO₂" means a plate-like sample prepared by peeling the outer whitish film with the titanium oxide photocatalyst layer and determining the peeled film. From the result shown in Fig. 8, it is understood that at a wavelength around 360 nm, the ultraviolet rays transmitted even in the sample of "inner and outer layers with TiO₂", and the "outer layer" caused the transmittance to be decreased in spite of the condition where the "outer layer" was a thin film.

Next, in order to evaluate the adhesive strength of the titanium oxide photocatalyst layer in the catheter formed with the titanium oxide photocatalyst layers after acid treating the silicone rubber catheter prepared as described above, the following model test was carried out.

After acid treating the surface of a silicone rubber substrate formed as a plate, a titanium oxide photocatalyst layer was formed on the surface (each layer, i.e., the adhesive layer and the titanium oxide photocatalyst layer, was formed by one-time coating). The conditions of the acid treatment and the coating of the adhesive layer and the titanium oxide photocatalyst layer were the same as those in the preparation of the aforementioned catheter (the film thickness of the formed titanium oxide photocatalyst layer: 1.5 µm). The substrate formed with the titanium oxide photocatalyst layer was bent around a core rod by an angle of 180 degrees for one second, based on the method of JIS-K-5400. The substrate was returned to the original state, and the occurrence of cracking and releasing away of the film of the titanium oxide photocatalyst layer was determined by observation. Although the number of bending times was increased from one time to 5, 30 and 50 times in the test, there occurred no cracking and releasing in any case. Further, after the bending test, although a cellophane tape was adhered and the examination for peeling the tape was carried out, the titanium oxide photocatalyst layer was not released. Furthermore, after the bending test with 50 repetitions, although the substrate was further bent 50 times in the contrary direction and was stretched and twisted as strongly as possible, the titanium oxide photocatalyst layer also was not released.

Further, although the above-described substrate was treated for 20 minutes in an autoclave at conditions of 120°C and 1 kgf/cm², the occurrence of cracking and release of the film was not recognized by observation.

Thus, the adhesive strength of the titanium oxide photocatalyst layer was extremely high against bending, stretching and twisting, and an excellent adhesive strength was exhibited even against the heating in the autoclave. In particular, when the surface of the titanium oxide photocatalyst layer was observed by an electron microscope, a great number of cracks (microcracks) were observed, the number of fine cracks increased in addition to relatively large cracks as the number of the bending times increased, but the above-described excellent close contact and adhesive properties in connection with the silicone rubber substrate side were maintained. Therefore, it is understood that such microcracks have a function for adequately absorbing the deformation force when the substrate having flexibility or elasticity receives a force repeatedly by bending, stretching or twisting, and this function serves to maintain the excellent close contact and adhesive properties.

Next, the antibacterial activity of the catheter with a titanium oxide photocatalyst layer was evaluated.

Three kinds of catheters were used: ① an all silicone balloon catheter of a kidney pelvis type, made of silicone rubber, " φ con"[brand name], ② a safety catheter made of silicone rubber, and ③ a suction catheter of silicone rubber, and as shown in Fig. 9, a 100-µL suspension of an Escherichia coli bacteria strain (E. coli, IF03301) 22 was stored in two kinds of catheters 21 with a titanium oxide photocatalyst layer and without the titanium oxide photocatalyst layer, both ends thereof were closed by caps 23, and from the outer side, ultraviolet rays with a wavelength of 360 nm were irradiated by an FLC black light fluorescent lamp (HOYA-SCOTT, UV LIGHT SOURCE UL200M).

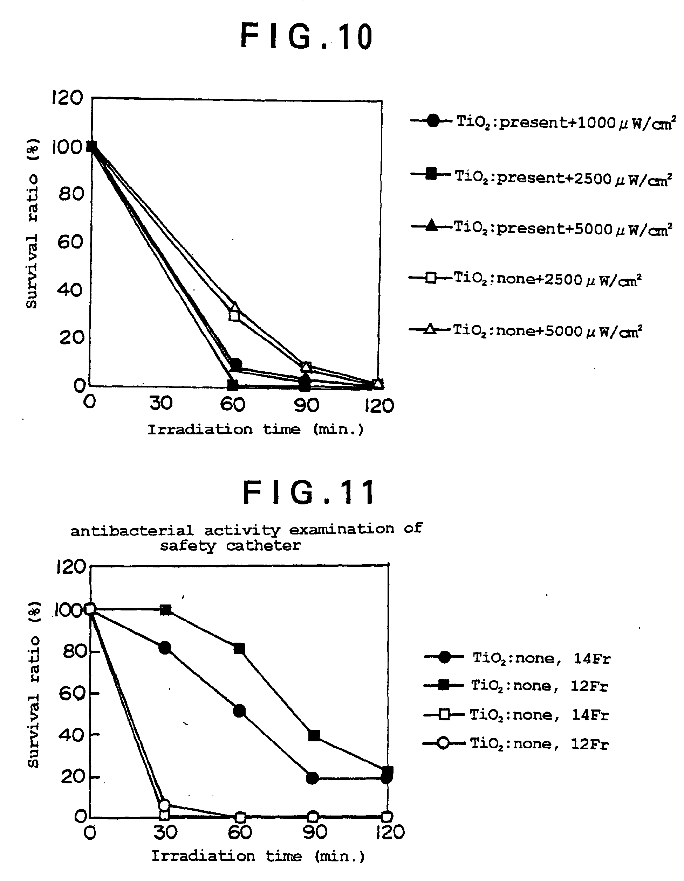

The result of the examination is shown in Fig. 10, wherein the 14 Fr catheters of the above-described ① was used and the irradiation intensity of the ultraviolet ray was controlled at 1000 µW/cm², 2500 µW/cm², and 5000 µW/cm². As shown in Fig. 10, although a relatively high bactericidal ratio (survival ratio at an irradiation time of 60 minutes: about 30%) was exhibited even in the case without the titanium oxide photocatalyst layer (TiO₂: none) at an irradiation intensity of not less than 2500 µW/cm² and the influence ascribed to the irradiation of the ultraviolet ray was exhibited, a sufficiently high bactericidal effect could be obtained even at an irradiation intensity of 1000 µW/cm² in the case with the titanium oxide photocatalyst layer (TiO₂: present).

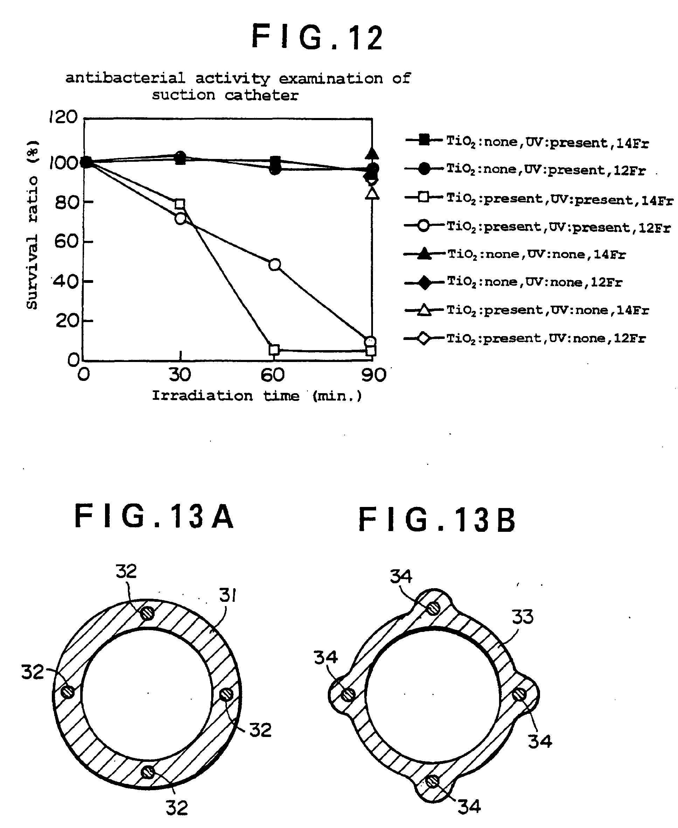

Figs. 11 and 12 show the examination results of the case of the safety catheters (14Fr, 12Fr) of the above-described ② and of the case of the suction catheters (14Fr, 12Fr). The irradiation intensity of the ultraviolet ray was controlled at 1100µW/cm² for the safety catheters and at 1000 µW/cm² for the suction catheters.

As is understood from Figs. 11 and 12, it was recognized that both kinds of catheters had excellent antibacterial activities ascribed to the titanium oxide photocatalyst layer. Further, in the safety catheters, bactericidal effect was exhibited even without the titanium oxide photocatalyst layer, and it is considered that the 14Fr catheter had a bactericidal efficiency higher than that of the 12Fr catheter, but as is evident from Fig. 12, it is understood that even in the case with the titanium oxide photocatalyst layer, unless the ultraviolet rays were irradiated, a desirable excellent antibacterial activity was not exhibited.

As described above, in any type of catheter, an excellent antibacterial activity ascribed to the titanium oxide photocatalyst layer could be recognized.

As the method for irradiating light to a catheter, although the light may be irradiated from the outside of the catheter as in the above-described examination, in order to irradiate the light to the inner and outer titanium oxide photocatalyst layers more efficiently, a light-guiding material may be provided to be buried in the tube body of the catheter. For example, as shown in Fig. 13A, a structure may be employed wherein light-guiding materials 32 are buried in a tube body 31 of a catheter formed with a titanium oxide photocatalyst layer on at least one of the inner and outer surfaces, and the light-guiding materials 32 extend in the longitudinal direction of the tube body 31 and can guide light toward the titanium oxide photocatalyst layer. Further, a cross-sectional structure shown in Fig. 13B may also be employed in accordance with the thickness a tube body 33 of a catheter and the diameter of a light-guiding material 34. The number of light-guiding materials 32, 34 is not particularly limited, and the positions and structures for burying them also are not particularly limited, and therefore, they may be appropriately designed depending upon the requirements.

Next, an examination was further carried out for confirming the effect in a case where an antibacterial substance in addition to the titanium oxide photocatalyst was carried out on a titanium oxide photocatalyst layer after the titanium oxide photocatalyst layer was formed. Silver was used as the antibacterial substance to be carried.

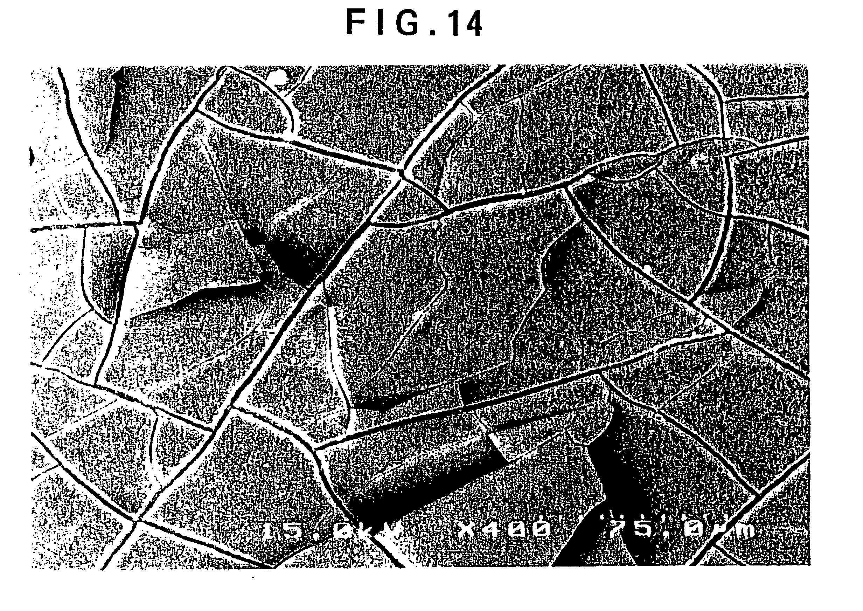

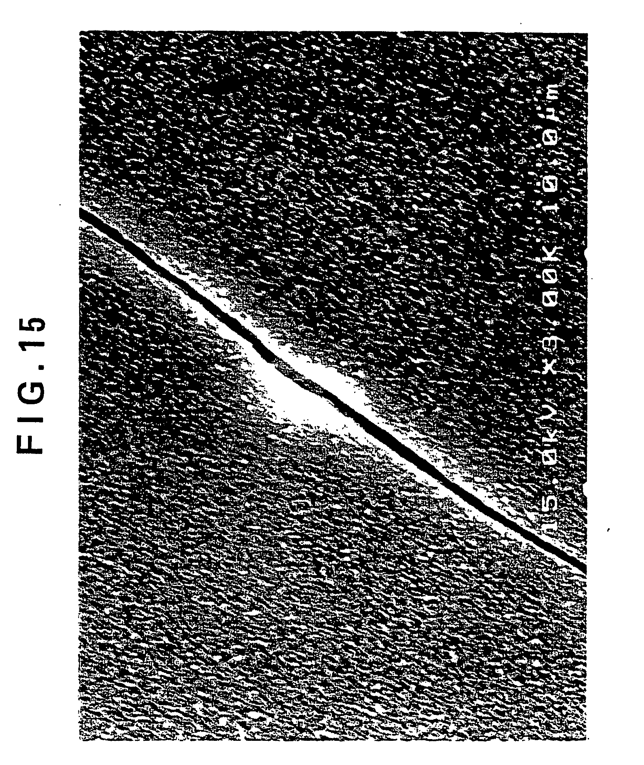

A silicone rubber suction catheter (size: 14Fr) formed with a titanium oxide photocatalyst layer by coating was soaked in 0.2 mM AgNO₃ solution (10% ethanol solution), and the silver was adsorbed and deposited on the titanium oxide photocatalyst layer by irradiating with ultraviolet rays (intensity: 1 mW/cm²) for 3, 5 and 7 minutes, respectively. Although the color of the silicone rubber substrate became brown by the irradiation of the ultraviolet rays and the silver carried was recognized even by visual observation, when it was observed by an electron microscope, the results shown in Figs. 14 and 15 were obtained, and in particular, it was recognized that the silver was effectively carried in microcracks of the titanium oxide photocatalyst layer and it vicinity (the portions observed to be whitish in Figs. 14 and 15). Since the catheter became deep in color as the irradiation time of the ultraviolet ray was longer, it is considered that the amount of precipitation of silver was great in such a catheter.

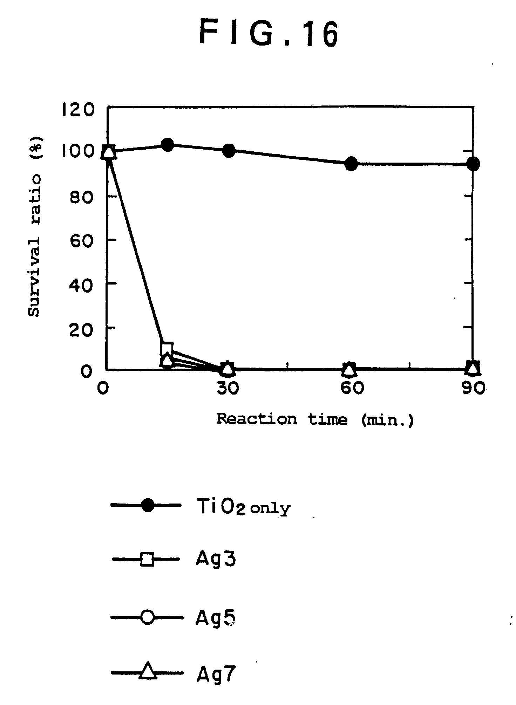

The examination for determining the antibacterial activity was carried out for catheter samples carried with this silver (a sample of the ultraviolet irradiation time for carrying silver of 3 minutes: Ag3, a sample of 5 minutes: Ag5, a sample of 7 minutes: Ag7) and for a catheter sample carried with no silver and on which only a titanium oxide photocatalyst layer was formed by coating (Ag0). The result of the examination is shown in the following Table and Fig. 16. In the examination, the same suspension of coli bacteria strain (E. coli, IF03301) as that aforementioned was used and it was given at a condition of 150 µl spot, the examination was carried out in a dark place in order to determine the antibacterial activity ascribed to the carried silver, and the survival ratio was determined.

| Survival ratio (%) | | | | | |
|---|---|---|---|---|---|
| Sample | Reaction time | | | | |
| | 0 min. | 15 min. | 30 min. | 60 min. | 90 min. |
| Ag0 | 100 | 102 | 100 | 94 | 94 |
| Ag3 | 100 | 9 | 1 | 0 | 0 |
| Ag5 | 100 | 3 | 1 | 0 | 0 |
| Ag7 | 100 | 5 | 2 | 0 | 0 |

As shown in the above Table and Fig. 16, it is understood that the catheters with carried silver on the titanium oxide photocatalyst layer (Ag3, Ag5, Ag7) sufficiently exhibited bactericidal effect even in the dark (even without irradiation of ultraviolet ray) as compared with one with no carried silver (Ag0). Further, because there is no great difference in effect between Ag3, Ag5 and Ag7, it is understood that the irradiation time of ultraviolet ray may be a fairly short period of time, and by only several minutes, the silver can be carried at an amount enough to exhibit a sufficient bactericidal effect.

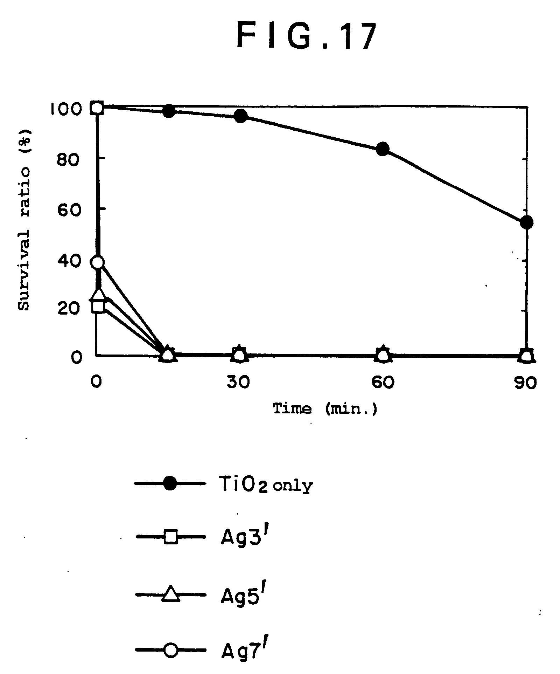

In order to determine the influence of the amount of carried silver to the antibacterial effect, silver was carried on a titanium oxide photocatalyst layer after the layer was formed on a thicker catheter (an all silicone Foley type, size: 26Fr) in a method similar to the above-described method. When an examination similar to the above-described examination was carried out with respect to the respective catheter samples (a sample of the ultraviolet irradiation time for carrying silver of 3 minutes: Ag3', a sample of 5 minutes: Ag5', a sample of 7 minutes: Ag7', a sample carried without carried silver and with only a titanium oxide photocatalyst layer: Ag0'), the result shown in Fig. 17 was obtained. As shown in Fig. 17, even in the case of the all silicone Foley type thick catheter, a high antibacterial effect could be recognized. It is understood that in this thick catheter, the amount of carried silver became fairly large because of the thickness, and sterilization was performed quickly in an extremely short time (in several seconds). From this result and the result shown in Fig. 16, it is understood that the speed of sterilization is in proportion to the amount of carried silver.

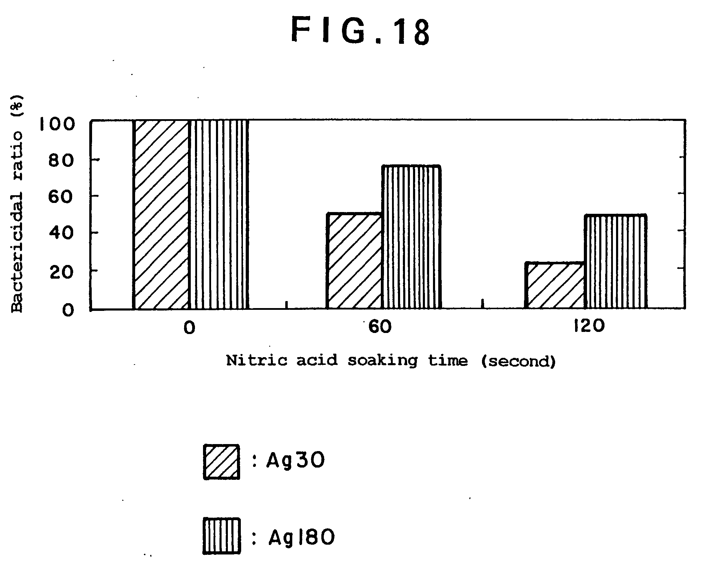

Further, the following examination was carried out in order to confirm whether the above-described antibacterial effect in the catheter with silver carried on the titanium oxide photocatalyst layer is ascribed to the carried silver or not. A titanium oxide photocatalyst layer was formed only on the inner surface of an all silicone Foley type catheter with a size of 18Fr, and silver was carried on the layer in a method similar to the above-described method to prepare samples (a sample of the ultraviolet irradiation time for carrying silver of 30 minutes: Ag30, a sample of 180 minutes: Ag180). The inner surfaces of these samples were soaked in nitric acid solution of 1 mol/L and the silver having been carried was removed by dissolution for 0 to 120 seconds. The amount of the carried silver decreased accompanying with the soaking time, and how the antibacterial effect was changing at that time was determined. The evaluation was carried out by an examination similar to the aforementioned examination carried out in the dark, and it was determined as the bactericidal ratio after 20 minutes. The result is shown in Fig. 18.

As shown in Fig. 18, since the bactericidal ratio decreased as the nitric acid soaking time increased in both Ag30 and Ag180 samples, it was recognized that the antibacterial effect in these catheters was ascribed to silver.

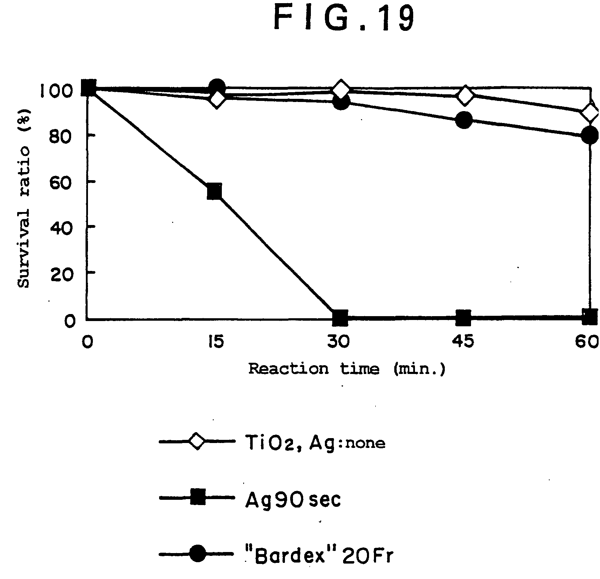

Furthermore, because the antibacterial activity ascribed to silver has been known, a comparison test was carried out between a catheter on the market on which silver was merely carried and a catheter with silver carried on a titanium oxide photocatalyst layer according to the present invention. In this comparison test, with respect to a sample of 20Fr silicone catheter formed merely with a titanium oxide photocatalyst layer (a sample of "Ag0sec), a sample deposited with silver on the titanium oxide photocatalyst layer by treatment of silver solution for 90 seconds (a sample of "Ag90sec") and a sample of a catheter with silver on the market ("Bardex" 20Fr), the same suspension of coli bacteria strain as that aforementioned was given at the condition of a 150-µL spot, and the test was carried out in the dark at conditions of bacterial solution treatment time (reaction time) of 0, 15, 30, 45 and 60 minutes, respectively. The result is shown in Fig. 19.

As shown in Fig. 19, in the conventional "Bardex" 20 Fr catheter, although a bactericidal effect is observed, it is weak (the bactericidal ratio at a reaction time of 60 minutes: about 20% [survival ratio: about 80%]). On the other hand, in the catheter with the titanium oxide photocatalyst layer and silver according to the present invention (Ag90sec), it became clear that the catheter had a strong bactericidal effect which was 100% bactericidal ratio (survival ratio: 0%) at a reaction time of 30 minutes. Therefore, as compared with the conventional catheter merely adhered with silver, the catheter with the titanium oxide photocatalyst layer and silver according to the present invention has a much higher antibacterial activity.

Further, an examination was carried out for forming a titanium oxide photocatalyst layer by the aforementioned method shown in Fig. 4. Silver was used as the antibacterial substance, and the titanium oxide photocatalyst layer was formed by the following method.

Silver ions (0.6 wt.%) were mixed into 200 mL paint for forming a photocatalyst layer NDC-100C, produced by Nihon Soda Corporation) by adding 0.2 g silver nitrate to the paint. While this paint for photocatalyst layer mixed with silver ions was stirred by a magnetic stirrer, the silver was precipitated by irradiating 1 mW/cm² ultraviolet ray for 5 days to prepare Ag-TiO₂ photocatalyst paint in which silver was carried on each titanium oxide particle. Using the prepared Ag-TiO₂ photocatalyst paint, first, a sample of an Ag-TiO₂ photocatalyst layer formed on a glass plate was formed, and when the color of the sample was determined on the glass plate, it exhibited a light yellow, and it was difficult to recognize the color of silver. Namely, silver was carried thinly and uniformly on the titanium oxide particles and a coating layer with an appearance nearly transparent was obtained. Thereafter, the above-described Ag-TiO₂ photocatalyst paint was coated onto the silicone rubber in a manner similar to that of the coating of the adhesive layer to form the photocatalyst layer.

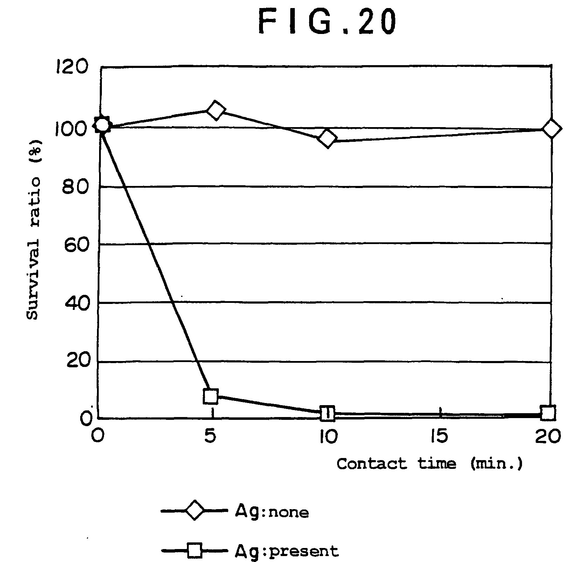

With respect to this sample formed with the Ag-TiO₂ photocatalyst layer and a sample carried with no silver, the bactericidal speeds (survival ratios) were determined at conditions similar to those shown in Fig. 16 (but, without irradiation of ultraviolet ray). The result is shown in Fig. 20. As shown in Fig. 20, it was recognized that the surface of the silicone rubber exhibited a high antibacterial activity even in the dark by the above-described method for forming the photocatalyst layer.

Although the explanation described hereinabove has been carried out mainly on catheters, the present invention can be applied not only to the catheters aimed at being inserted into or placed in human bodies but also to any tube for medical care comprising an elastomer tube body and having a requirement of antibacterial activity.

### Industrial Applications of the Invention

In the tube for medical care and the method for preparing the same according to the present invention, even if the tube comprises an elastomer and has flexibility, a titanium oxide photocatalyst layer can be fixed on the tube extremely firmly, and an excellent antibacterial activity ascribed to the photocatalyst layer can be exhibited. Further, when, an antibacterial substance is carried on the titanium oxide photocatalyst layer, a further excellent antibacterial activity can be exhibited, and at the same time, an excellent antibacterial activity can be given even in the dark. Therefore, such a tube for medical care according to the present invention can be applied broadly to a catheter, an endoscope and other tubes for medical care, and is remarkably useful in the medical field.

## Claims

1. A tube for medical care comprising:
a tube body comprising an elastomer; and
a photocatalyst layer formed on the surface of said tube body, said photocatalyst layer containing titanium oxide and having a great number of microcracks.

2. The tube for medical care according to claim 1, wherein an antibacterial substance is carried on said photocatalyst layer containing titanium oxide.

3. The tube for medical care according to claim 1, wherein said antibacterial substance is silver.

4. The tube for medical care according to claim 1, wherein said antibacterial substance is carried on the surfaces of titanium oxide particles forming said photocatalyst layer.

5. The tube for medical care according to claim 4, wherein said antibacterial substance is silver.

6. The tube for medical care according to claim 1, wherein an adhesive layer is interposed between at least one of the inner and outer surfaces of said tube body and said photocatalyst layer containing titanium oxide, and said photocatalyst layer and said adhesive layer, are formed substantially as an integral layer.

7. The tube for medical care according to claim 1, wherein a light-guiding material, extending in the longitudinal direction of said tube body and capable of guiding light toward said photocatalyst layer containing titanium oxide, is buried in said tube body.

8. The tube for medical care according to claim 1, wherein said tube body is made of a silicone rubber.

9. The tube for medical care according to claim 1, wherein the thickness of said photocatalyst layer is in the range of 10 nm to 10 µm.

10. The tube for medical care according to claim 1, wherein said tube is a catheter to be inserted into or placed in a human body.

11. The tube for medical care according to claim 1, wherein said tube is an endoscope.

12. A method for preparing a tube for medical care comprising the steps of:
treating the surface of a tube body comprising an elastomer with an acid; and
forming a photocatalyst layer containing titanium oxide on said treated surface of said tube body by coating, and at the same time, generating a great number of microcracks in said photocatalyst layer.

13. The method for preparing a tube for medical care according to claim 12, wherein an adhesive layer is coated after said acid treatment of the surface of said tube body, and said photocatalyst layer containing titanium oxide is coated on said adhesive layer.

14. The method for preparing a tube for medical care according to claim 12, wherein an antibacterial substance is carried on said photocatalyst layer after forming said photocatalyst layer.

15. The method for preparing a tube for medical care according to claim 14, wherein silver is used as said antibacterial substance.

16. The method for preparing a tube for medical care according to claim 12, wherein an antibacterial substance is carried on titanium oxide particles, and a photocatalyst layer containing said titanium oxide particles carried with said antibacterial substance is coated.

17. The method for preparing a tube for medical care according to claim 16, wherein silver is used as said antibacterial substance.

18. The method for preparing a tube for medical care according to claim 12, wherein said tube for medical care is a catheter to be inserted into or placed in a human body.

19. The method for preparing a tube for medical care according to claim 12, wherein said tube for medical care is an endoscope.
